# EUROPEAN PATENT APPLICATION

(11) **EP 1 774 978 A1**
(43) Date of publication of application: **18.04.2007**
(21) Application number: 05751331.9
(22) Date of filing: 03.06.2005
(51) Int. Cl.: A61K 49/00, A61K 9/08, A61K 47/36

(54) **APPLICATION OF HYALURONIC ACID TO SENTINEL LYMPH NODE IDENTIFICATION**

(30) Priority: 03.06.2004 JP 2004165508
(71) Applicant: Kitayama, Joji, Bunkyo-ku, Tokyo 1120006 (JP)
(72) Inventor: Kitayama, Joji, Bunkyo-ku, Tokyo 1120006 (JP)
(74) Representative: Keller, Günter
(86) International application number: PCT/JP2005/010635
(87) International publication number: WO 2005/117995

(57) **Abstract**

The present invention provides an accurate, convenient, and safe agent and method for detecting sentinel lymph nodes. The present invention enables the accurate, convenient, and safe detection of sentinel lymph nodes through the use of a mixed component of a coloring component and a hyaluronic acid component

## Description

### TECHNICAL FIELD

The present invention relates to an agent for sentinel lymph node biopsy whose main component is a mixed component of a coloring component and a hyaluronic acid component and also relates to a method for detecting a sentinel lymph node using the detection agent.

### BACKGROUND ART

Metastasis of a malignant tumor include metastasis via blood circulation and via lymphatics. In the case of hematogeneous metastasis, cancer cells are carried to various organs through bloodstream and form metastatic foci. Metastasis via lymphatic circulation, on the other hand, occurs in the area of lymphatic vessels draining the primary tumor. However, it is impossible to accurately recognize a metastatic lymph node prior to and during surgery, and cancer recurs if a metastatic lymph nodes remain in the body. The general rule in the surgical treatment of cancer has therefore been extirpation of the primary focus and the associated lymph nodes. However, the surgical removal of the associated lymph nodes as a group often impaiers the quality of life (QOL) of the patient, and the significance of lymphadenectomy has been regarded as questionable in cases of early stage cancer which is rarely associated with nodal metastasis. However, because preoperative diagnosis of lymph node metastasis is not accurate and the failure to remove the metastatic lymph nodes is thought to be fatal for patients, classical lymphadenectomy with curative intent cannot be omitted at the present time in cancer surgery.

If, however, lymph flow from the primary cancer can be accurately recognized, it is possible to predict which lymph node will develop the initial metastasis. Lymph nodes are located along the a lymphatic vessel network, and carry out a function, by controlling local immune response, of efficiently removing toxins and microorganisms which have invaded the body. The sentinel lymph node (SNL) is the lymph node that initially receives the lymph flow from the primary site of a malignant tumor, and is also known as the guard lymph node or sentry lymph node. Cancer cells, after have invaded a lymphatic vessel, are carried to this sentinel lymph node by the lymphatic flow. However, because they raised an immune response there, it is considered to be difficult for them to pass through this lymph node. Therefore, the metastasis of cancer via lymphatic circulation is thought to be developed at the SLN. Thus, it is theoretically applicable to improve the patient's QOL to omit the conventional lymphadenectomy when we can determine that the metastasis in SLN is not present, although if cancer metastasis to the SLN is recognized, the conventional lymphadenectomy should be carried out This is the general idea of sentinel node navigation surgery and it becomes to be applied to clinical surgery in the areas of breast cancer and malignant melanoma in these years.

The most significant problem in the clinical application of this sentinel node navigation surgery is to establish a highly reliable method for identifying the SLN during surgery. For this purpose, a radioisotope (RI) procedure, which uses a radioisotopic colloid (RI), and a dye procedure are mainly conducted on trial.

The RI procedure has the advantage that the colloid particles, due to their relatively large size, have a distinct tendency to be retained in the lymph node. However, the RI method requires special facilities and a gamma probe for detection, and it is not easy to come into wide use. Another drawback is that the use of a radioisotope results in exposure to radiation. The SLN detection rate with the RI procedure is relatively high, with an identification rate of 90 to 95%, that is not still satisfactory in clinics.

On the other hand, the dye procedure is a convenient procedure in which a blue dye, such as indigo carmine or patent blue, is injected in the region of the primary tumor site, and the lymph flow and the initially stained lymph node are detected by means of coloration. Since the dye procedure does not use a radioisotope, it offers the advantage of no exposure to radiation. However, due to the very rapid outflow of the colorant from the lymphatic vessels, the SLN must be identified in a very short period of time (within about 15 minutes) and the identification rate generally tends to be low at 75 to 85%. This can be considered a fatal drawback because one considers that the early stage cancer, which is a target, is almost 100% cured by conventional lymphadenectomy.

Thus, development of a more accurate, more convenient, and safer method for detecting SLNs, particularly with respect to organs that have an anatomically complex lymphatic system, is required.

Hyaluronic acid is a biological material (a polysaccharide) that was isolated from the vitreous body of bovine eyes by K. Meyer in 1934 and was known as a major component of extracellular matrix from a long time ago. Hyaluronic acid does not have interspecies differences in its chemical or physical structure and human also has a metabolic system, and in addition, it can be regarded as a completely safe biomaterial from the perspectives of immunity and toxicity. Hyaluronic acid is already in clinical application as pharmaceuticals such as a therapeutic agent for joint diseases (Artz (trade name), manufactured by Seikagaku Corporation and distributed by Kaken Pharmaceutical Co., Ltd.; Subenil (trade name), manufactured and distributed by Chugai Pharmaceutical Co., Ltd.). It is also known that hyaluronic acid is highly transferable into the lymphatic system and that local lymph nodes are thought to be the main sites for hyaluronic acid degradation. However, there have not been reported that hyaluronic acid can be applied to sentinel node navigation surgery.

### DISCLOSURE OF THE INVENTION

An object of the present invention is to provide an accurate and convenient agent for detecting sentinel lymph nodes and an accurate, convenient, and safe method for detecting sentinel lymph nodes.

The present inventors have carried out extensive investigations in order to solve the problems and have discovered that sentinel lymph nodes can be accurately, conveniently, and safely detected using a mixed component of a coloring component and a hyaluronic acid component, and have come to accomplish the present invention.

That is, the present invention relates to an agent for sentinel lymph node detection whose main component is a mixed component of a coloring component and a hyaluronic acid component. The present invention further relates to a method for detecting a sentinel lymph node in which a mixed component of a coloring component and a hyaluronic acid component is administered at the primary tumor site of a cancer patient. In particular, the hyaluronic acid component used here is preferably sodium hyaluronate. Weight-average molecular weight of the hyaluronic acid component is preferably in the range of from 600,000 to 6,000,000 daltons and more preferably is in the range of from 1,900,000 to 6,000,000 daltons. The detection agent according to the present invention is preferably in the form of an aqueous solution, and preferably is an injectable formulation. The coloring component used in the agent for sentinel lymph node detection according to the present invention preferably has a dark color in order to provide good visibility of the subject site. Moreover, the coloring component preferably is in the form of microparticles in order to achieve longer residence time in the sentinel lymph node. The coloring component is therefore preferably selected from blue colorants, green colorants, magnetic iron materials, colloidal iron, and colored microparticles.

Sentinel lymph nodes can be accurately, conveniently and safely detected using the agent for sentinel lymph node detection according to the present invention.

### BRIEF DESCRIPTION OF THE DRAWINGS

Figure 1 shows the coloring effect from the associated lymphatic vessels to the sentinel lymph node, at 20 minutes after the application of detection agent comprising patent blue and hyaluronic acid.
Figure 2 shows a sentinel lymph node at 3 hours after the injection of a ferumoxides injection solution and hyaluronic acid. The diagram on the left shows the condition prior to dissection of the sentinel lymph node, while the diagram on the right shows the sentinel lymph node after dissection.
Figure 3 shows a sentinel lymph node at 2 days after the injection of a ferumoxides injection solution and hyaluronic acid. The diagram on the left shows the condition prior to dissection of the sentinel lymph node, while the diagram on the right shows a photomicrograph after extirpation of the sentinel lymph node.

### BEST MODE FOR CARRYING OUT THE INVENTION

Hereinafter, the present invention is described more specifically.

The agent for sentinel lymph node detection according to the present invention is the agent whose main component is a mixed component of a coloring component and a hyaluronic acid component. A characteristic feature of the present invention resides in the fact that the residence time of the coloring component in the target sentinel lymph node is extended by adding the hyaluronic acid component to the coloring component. The coloring component in the detection agent according to the present invention can provide coloration at a level that enables discrimination of a sentinel lymph node with naked eyes from other lymph nodes preferably for at least 1 hour, more preferably for at least 2 hours, and even more preferably for at least 24 hours.

The "hyaluronic acid component" of the present invention encompasses those substances generally called hyaluronic acid and may be, for example, a high molecular weight polysaccharide containing glucuronic acid and N-acetylglucosamine as repeat units, and also includes hyaluronic acid derivatives in which a portion of the hyaluronic acid has been derivatized, as well as salts thereof. There are no particular limitations. It can be, for example, a polymer of two saccharides comprising glucuronic acid and N-acetylglucosamine, with an average molecular weight of from 50,000 to 10,000,000 daltons. The hyaluronic acid salts are not particularly limited and include, for example, sodium, potassium, calcium, aluminum, zinc, iron, ammonium, and tetrabutylammonium salts. Specific examples of hyaluronic acid, its salts, and mixtures thereof include a product named Suvenyl (trademark, manufactured and distributed by Chugai Pharmaceutical Co., Ltd.), a product named Artz (trademark, manufactured by Seikagaku Corporation and distributed by Kaken Pharmaceutical Co., Ltd.), and a product named Opegan (trademark, manufactured by Seikagaku Corporation and distributed by Santen Pharmaceutical Co., Ltd.). "Hyaluronic acid derivatives" in the present invention denote substances having a hyaluronic acid backbone derived from hyaluronic acid. The hyaluronic acid derivatives are not particularly limited and include, for example, substances in which at least one carboxyl group in hyaluronic acid is esterified (for example, benzyl-esterified hyaluronic acid (product name: Hyaff (trademark), Fidia Advanced Biopolymers)), further polymerized substances by crosslinking hyaluronic acids with formaldehyde (for example, product name: Synvisc (trademark), Biomatrix), and acetylated hyaluronic acid in which at least one hydroxyl group in hyaluronic acid is acetylated. Sodium hyaluronate, which is frequently used as a pharmaceutical product, is preferred among them. Material with a weight-average molecular weight of from 600,000 to 6,000,000 daltons is more preferred, and material with a weight-average molecular weight of from 1,900,000 to 6,000,000 daltons is particularly preferred among these.

Hyaluronic acid and its pharmaceutically acceptable salts can be produced using various known methods, such as a method by extraction from biological sources such as the rooster comb and pig subcutaneous tissue or a method by biofermentation, or can also be obtained by purchasing commercial products (from, for example, Denki Kagaku Kogyo Kabushiki Kaisha, Shiseido Co., Ltd., or Seikagaku Corporation).

Any component can be used as the coloring component, provided that it is safe for living organisms and has good visibility.

Because, the color of the biological matrix that forms the background is yellow, from the standpoint of visibility, the coloring component is preferably a material having a dark color (for example, a greenish color such as green and dark green; a brownish color such as reddish brown, deep brown, dark reddish brown, and dark brown; a bluish color such as blue, navy blue, and dark navy blue; and black). The bluish colors, which can be clearly distinguished from the background, are particularly preferred.

From the standpoint of retention, the coloring component is preferably in the form of microparticles. The maximum size of the microparticles is preferably from 10 nm to 10,000 nm.

Specific examples of such coloring components are as follows: blue dyes such as methylene blue, indigo carmine, isosulphan blue, and patent blue, which are already used in SLN identification method (dye procedure); green dyes such as indocyanine green; magnetic iron materials such as ferumoxides (product name: Feridex (trademark), manufactured and distributed by Eiken Chemical Co., Ltd., distributed by Tanabe Seiyaku Co., Ltd.) and ferucarbotran (product name: Resovist Inj. (trademark), manufactured and distributed by Nihon Schering Kabushiki Kaisha), which are used as liver contrast media for MRI; colloidal iron such as chondroitin sulfate · iron colloid (product name: Blutal (trademark), manufactured and distributed by Dainippon Pharmaceutical Co., Ltd., distributed by Chugai Pharmaceutical Co., Ltd.), chondroitin sulfate · iron colloid (product name: Atofen Inj. (trademark), manufactured and distributed by Uji Pharmaceutical Co., Ltd.), and cideferron (product name: Ferricon (trademark), manufactured and distributed by Nippon Zoki Pharmaceutical Co., Ltd.), which are used as iron agents for intravenous injection; and colored microparticles such as pigment ink, which is used, for example, in toners for inkjet printers.

As a coloring component used in the present invention, for example, a dispersion of microparticulate form having a suitable particle size which is prepared by mixing a dispersing medium and a resin into a colored material can be used. The residence time in the sentinel lymph node can be prolonged by using such a microparticulate dispersed coloring component. As a carrier fluid used as a dispersing medium, for example, cyclohexane, n-hexane, n-heptane, n-nonane, n-octane, isooctane, isododecane, ligroin, and mixtures thereof can be used. As a resin, for example, polystyrene resins, polyacrylic resins, polyester resins, polyurethane resins, epoxy resins, polyvinylbutyral resins, polyolefin resins, silicone resins, and polycarbonate resins, etc. can be used. The colored material present in this microparticulate dispersed coloring component can be, in addition to the colorants described above, an inorganic pigment such as a carbon black such as Printex V, Printex U, Printex G, Special Black 15, Special Black 4, and Special Black 4-B (the preceding are products of Degussa); Mitsubishi #44, Mitsubishi #30, Mitsubishi MR-11, and Mitsubishi MA-100 (the preceding are products of Mitsubishi Chemical Corporation); Raven 1035, Raven 1252, and New Spectra II (the preceding are products of Columbia Carbon); and Regal 400, Regal 600, Black Pearl 900, Black Pearl 1100, and Black Pearl 1300 (the preceding are products of Cabot Corporation), titanium oxide, or zinc oxide; or an organic pigment such as Phthalocyanine Blue, Phthalocyanine Green, Sky Blue, Rhodamine Lake, Malachite Green Lake, Methyl Violet Lake, Peacock Blue Lake, Naphthol Green B, Naphthol Green Y, Naphthol Yellow S, Naphthol Red, Lithol Fast Yellow 2G, Permanent Red 4R, Brilliant Fast Scarlet, Hansa Yellow, Benzidine Yellow, Lithol Red, Lake Red C, Lake Red D, Brilliant Carmine 6B, Permanent Red F5R, Pigment Scarlet 3B, Indigo, Thioindigo, Oil Pink, and Bordeaux 10B These resin-containing coloring components can be prepared, for example, by dispersing 50 to 98 weight parts dispersion medium, 40 to 0.5 weight parts colored material, and 0.5 to 45 weight parts resin, and adding a polarity control agent and dispersing agent as needed, to a particle size of about 0.01 to 5 µm using an attritor, ball mill, or pin mill, etc.

The method for mixing the coloring component and the hyaluronic acid component may be any method that is capable of providing uniform and thorough mixing (or dispersion). For example, when a blue colorant or green colorant is used as the coloring component, the mixing can be accomplished by preparing a 1% w/v aqueous solution of the hyaluronic acid component and a 5% w/v aqueous solution of the coloring component and mixing these two solutions in a desired ratio. When a magnetic iron material or iron colloid is used as the coloring component, the mixing can be accomplished by preparing a 1% w/v aqueous solution of the hyaluronic acid component and an aqueous solution of the coloring component having an iron concentration of 0.4% w/v to 3% w/v and mixing these two solutions in a desired ratio. When colored microparticles are used as the coloring component, the mixing can be accomplished by preparing a 1% w/v aqueous solution of the hyaluronic acid component and a liquid pigment ink, which is commercially available as a toner for inkjet printers, etc., and mixing these two solutions in a desired ratio. In all of these cases, the mixing ratio, expressed as volumetric ratio of the aqueous hyaluronic acid component solution : aqueous coloring component solution, is 1 : 1 to 1, 16, preferably 1 : 2 to 1 : 8, and particularly preferably 1 : 4.

The agent for sentinel lymph node detection according to the present invention is preferably formulated as an aqueous solution, and particularly preferably is formulated as an injectable formulation. In order to formulate as an injectable formulation, for example, it may be dissolved in a desired concentration in physiological saline or phosphate-buffered physiological saline. At this point, the solution may be adjusted to a desired pH by adding acid or base as needed. The solution can also be adjusted to a desired salt concentration by adding, for example, such as an inorganic salt, such as a monovalent metal salt, e.g., a sodium or potassium salt, etc., or a divalent metal salt, e.g., a magnesium, calcium, or manganese salt, etc. A stabilizer and so forth may also be added as desired. The thus-prepared solution, in which the agent for sentinel lymph node detection according to the present invention is dissolved, may be distributed as a form of pre-filled injection syringe which is, for example, a disposable syringe barrel.

When administering the agent for sentinel lymph node detection according to the present invention as an injectable formulation, the hyaluronic acid concentration is adjusted to 0.01% w/v to 2% w/v, preferably 0.05% w/v to 1% w/v, and particularly preferably 0.1% w/v to 0.5% w/v. The colorant component concentration is adjusted to 2.5% w/v to 5% w/v when a blue colorant or green colorant is used as the coloring component, and to an iron concentration of 0.2% w/v to 3% w/v when a magnetic iron material or an iron colloid is used as the coloring component. This injectable formulation can be administered, at 0.5 mL to 3 mL per administration, into the region of the primary tumor site. This administration may be divided into a plurality of times, and the dose can be increased or decreased as appropriate to give a particular optimal dose taking into consideration the physician's instructions, the particular patient, the site of administration, the molecular weight of the hyaluronic acid used as an ingredient, the type of colorant component, and so forth.

### EXAMPLES

Hereinafter, preferred examples of the present invention will be described in more detail, however, the present invention is not limited to these examples.

Sodium hyaluronate with an average molecular weight of from 1,900,000 to 2,500,000 (from Chugai Pharmaceutical Co., Ltd.) was used as the hyaluronic acid component in the following examples.

### Example 1

### Detection of the sentinel lymph node with a mixed component of blue colorant and hyaluronic acid component

### (1) Preparation of a mixed component of blue colorant and hyaluronic acid component

Preparation was carried out by mixing a 1% w/v aqueous solution of sodium hyaluronate and a 5% w/v aqueous patent blue solution prepared by dissolving patent blue (from Wako Pure Chemical Industries, Ltd.) in injection-grade water at proportions of 1 : 1, 1 : 2, 1 : 4, 1: 8, or 1 : 16 as the volumetric ratio.

### (2) Sentinel lymph node detection using the mixed component of blue colorant and hyaluronic acid component

A pig was laparotomized under general anesthesia and the mixture of the sodium hyaluronate and the patent blue prepared in (1) above and patent blue alone as a control were each injected at two locations which were in the tissues (submucosal) of the stomach and small intestine at 0.5 mL each, and migration from the lymphatic vessels to the lymph nodes was monitored for 3 hours after injection based on the changes in color under laparotomy. The abdomen was then closed and a laparotomy was again performed after 1 to 2 days and migration in the tissues was observed at the time. In addition, the colored lymph nodes were investigated histologically after 3 hours and after 24 hours.

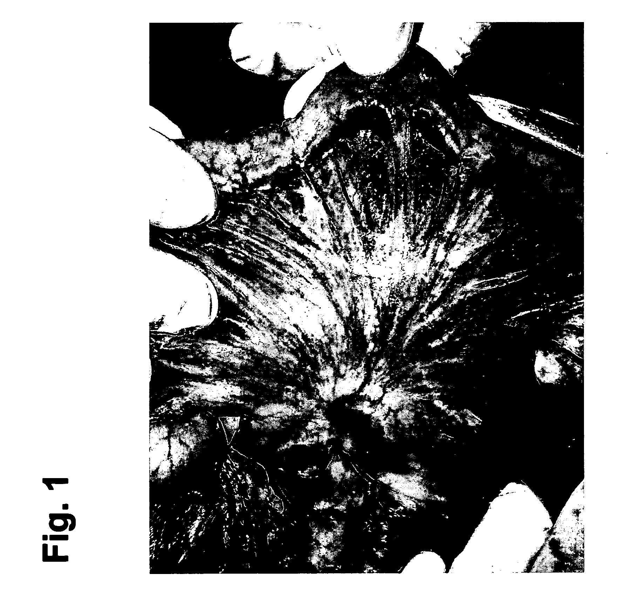

In the case of patent blue alone, the lymphatic vessels of the stomach wall and small intestine wall were dyed at several minutes after injection, and it could be confirmed that the patent blue quickly arrived at the lymph nodes; however, it had almost completely disappeared from the associated lymphatic system within 2 hours. In contrast, in the case of the mixtures of hyaluronic acid at greater than or equal to 1 : 4 as the volumetric ratio versus patent blue, while there was no substantial change in time until staining of the lymphatic system, the color was retained only within the associated lymphatic vessels and the initial lymph node (SLN) even at 2 hours after injection, and staining of lymphnodes located at further wide-area was not observed. The SLN began to be stained from the area peripheral to the inflow region for the afferent lymphatic vessels, and after 20 minutes, it was identified as the sentinel lymph node which was stained only a portion of the SLN (Figure 1). Thereafter, the color of the SLN gradually faded, but even at the time of closure of the abdomen and at the time of the relaparotomy (24 hours after injection), the SLN could be discriminated from other lymph nodes with naked eyes.

### Example 2

### Detection of the sentinel lymph node using a mixture of magnetic iron material and hyaluronic acid component

### (1) Preparation of a mixture of magnetic iron material and hyaluronic acid component

Preparation was carried out by mixing a 1% w/v aqueous solution of sodium hyaluronate and a ferumoxides injection solution (from Eiken Chemical Co., Ltd.) having an iron concentration of 1.12% w/v at proportions of 1 : 1, 1 : 2, 1 : 4, 1 : 8, or 1 : 16 as the volumetric ratio.

### (2) Sentinel lymph node detection using the mixture of magnetic iron material and hyaluronic acid component

A pig was laparotomized under general anesthesia and the mixture of the sodium hyaluronate and the ferumoxides injection solution prepared in (1) above and the ferumoxides injection solution alone as a control were each injected at two locations which were in the tissues (submucosal) of the stomach and small intestine at 0.5 mL each, and migration from the lymphatic vessels to the lymph nodes was monitored for 3 hours after injection based on the changes in color under laparotomy. The abdomen was then closed and a laparotomy was again performed after 1 to 2 days and migration in the tissues was observed at the time. In addition, the colored lymph nodes were investigated histologically after 3 hours and after 24 hours.

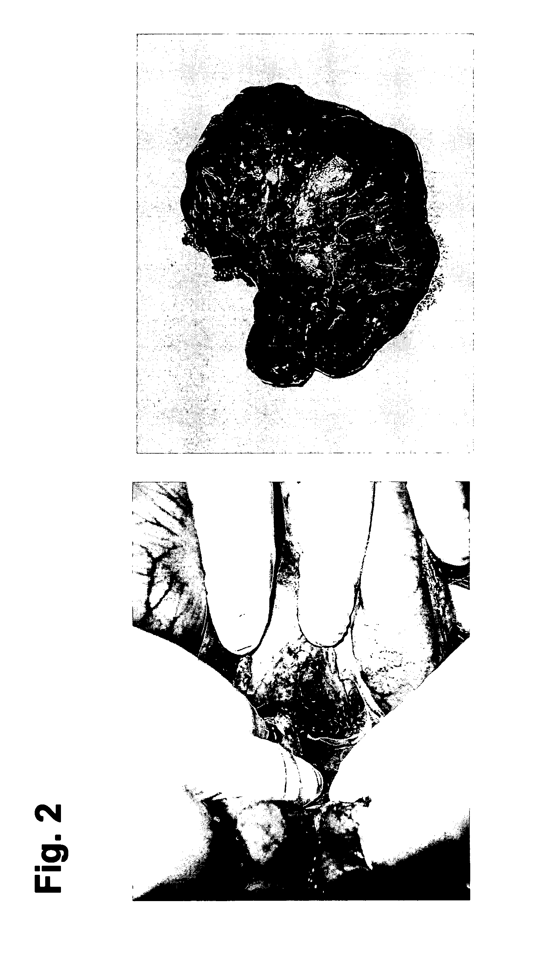

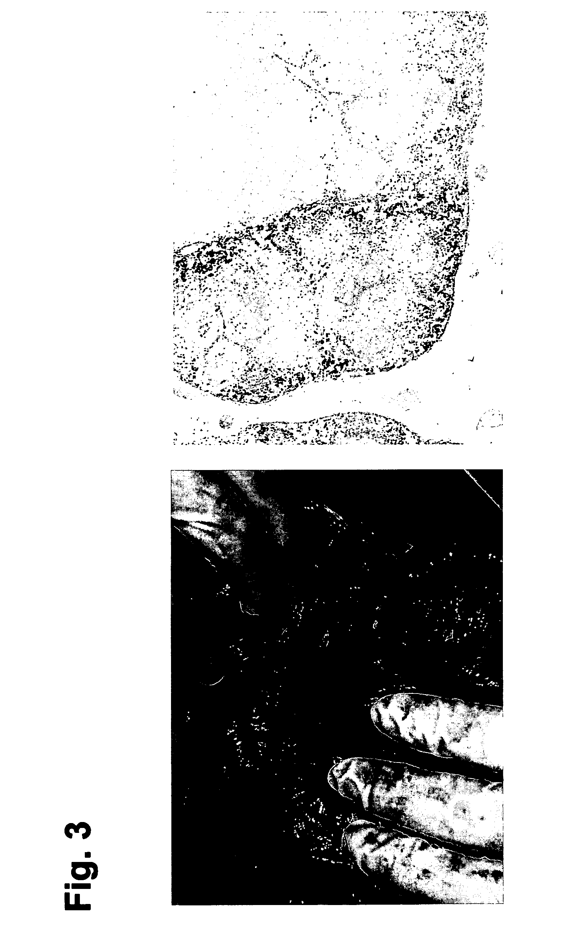

In the case of the ferumoxides injection solution alone, the SLN began to stain blackish brown from the lymphatic vessels from the peripheral region in about the same elapsed time as for patent blue alone and was identified as the SLN even after 3 hours. After a day, however, other lymph nodes were also stained blackish color and the SLN could not be distinguished from the other lymph nodes. However, in the case of the mixtures of sodium hyaluronate and ferumoxides injection solution at volumetric ratios of greater than or equal to 1 : 8, while a substantial difference in the time until staining of the SLN from the lymphatic system was not observed, the speed of the color change within the SLN was clearly slowed, and only a portion of the SLN was clearly stained even after 3 hours (Figure 2). Furthermore, even after 24 and 48 hours, the SLN alone could be clearly distinguished from other lymph nodes as a blackish brown lymph node (Figure 3, left).

With regard to this phenomenon of retardation of lymphatic system diffusion, no significant difference in the elapsed time was observed among the mixtures of sodium hyaluronate and ferumoxides injection solution at proportions of 1 : 1, 1 : 2, and 1 : 4 as the volumetric ratio. However, retardation of lymphatic system diffusion was not clearly observed for mixtures of sodium hyaluronate and ferumoxides injection solution at proportions of less than or equal to 1 : 16 as the volumetric ratio. Mixtures of sodium hyaluronate and ferumoxides injection solution at proportions of greater than or equal to 1 : 2 as the volumetric ratio, however, were somewhat difficult to inject within the mucosa due to a strong resistance at the time of injection.

When lymph node sections were subjected to iron staining, the magnetic iron material (ferumoxides) was observed to be actively incorporated in phagocytes, such as macrophages and so forth, within the marginal sinus and the lymph sinuses of the medulla. Because these intracellular iron particles are remained localized unless the phagocytes migrate, it is considered as a cause of long-term maintenance of lymph node staining (Figure 3, right).

### Example 3

### Detection of the sentinel lymph node using a mixture of magnetic iron material and hyaluronic acid component

### (1) Preparation of a mixture of magnetic iron material and hyaluronic acid component

Preparation was carried out by mixing a 1% w/v aqueous solution of sodium hyaluronate and ferucarbotran injection solution (from Nihon Schering Kabushiki Kaisha) having an iron concentration of 2.79% w/v at proportions of 1 : 1, 1 : 2, 1 : 4, 1 : 8, or 1 : 16 as the volumetric ratio.

### (2) Sentinel lymph node detection using the mixture of magnetic iron material and hyaluronic acid component

A pig was laparotomized under general anesthesia and the mixture of the sodium hyaluronate and the ferucarbotran injection solution prepared in (1) above and the ferucarbotran injection solution alone as a control were each injected at two locations which were in the tissues (submucosal) of the stomach and small intestine at 0.5 mL each, and migration from the lymphatic vessels to the lymph nodes was monitored for 3 hours after injection based on the changes in color under laparotomy. The abdomen was then closed and a laparotomy was again performed after 1 to 2 days and migration in the tissues was observed at the time. In addition, the lymph nodes were investigated histologically after 3 hours and after 24 hours.

Transition course to the lymphatic system was about the same as for the use of ferumoxides in Example 2, and it was confirmed that it continued in a single lymph node even after 24 hours.

### Example 4

### Detection of the sentinel lymph node using a mixed component of colored microparticles and hyaluronic acid component

### (1) Preparation of a mixed component of colored microparticles and hyaluronic acid component

Preparation was carried out by mixing a 1% w/v aqueous solution of sodium hyaluronate and a liquid cyan pigment ink (from Fuji Film Business Supply Co., Ltd.) that is commercially available as a toner for the "Kaleida" (trademark, from Fuji Film Business Supply Co., Ltd.) series of inkjet printers at a proportion of 1 : 4.

### (2) Sentinel lymph node detection using the mixed component of colored microparticles and hyaluronic acid component

A pig was laparotomized under general anesthesia and the mixture of the sodium hyaluronate and the liquid cyan pigment ink prepared in (1) above was injected at two locations which were in the tissues (submucosal) of the stomach and small intestine at 0.5 mL each, and migration from the lymphatic vessels to the lymph nodes was monitored for 3 hours after injection based on the changes in color under laparotomy. The abdomen was then closed and a laparotomy was again performed after 1 to 2 days and migration in the tissues was observed at the time. In addition, lymph node was investigated histologically after 3 hours and after 24 hours.

Strong staining was obtained as a result and the SLN could be clearly detected even after 24 hours.

According to the description above, the agent for sentinel lymph node detection of the present application, whose main component is a mixed component of a coloring component and a hyaluronic acid component, did not exhibit much retardation of time until staining of the associated lymphatic vessels in comparison with the use of the colorant component alone, but the color migration rate in the lymphatic system was substantially reduced and the initially stained lymph node could be clearly discriminated from other lymph nodes for a period of at least 1 to 2 hours after injection. It is thought that this time course provides sufficient for SLN detection during surgery.

### INDUSTRIAL APPLICABILITY

An accurate, convenient, and safe agent for detecting sentinel lymph nodes can be provided by using a mixed component of a coloring component and a hyaluronic acid component as the main component of the agent.

## Claims

1. An agent for a sentinel lymph node detection, wherein a main component of said agent is a mixed component of a coloring component and a hyaluronic acid component.

2. The agent for sentinel lymph node detection according to claim 1, which is in the form of an aqueous solution.

3. The agent for sentinel lymph node detection according to claim 1, which is an injectable formulation.

4. The agent for sentinel lymph node detection according to claim 1, wherein said coloring component has a dark color.

5. The agent for sentinel lymph node detection according to claim 1, wherein said coloring component is in a microparticulate form.

6. The agent for sentinel lymph node detection according to claim 1, wherein said coloring component is a blue colorant, a green colorant, a magnetic iron material, an iron colloid, or colored microparticles.

7. The agent for sentinel lymph node detection according to claim 1, wherein said hyaluronic acid component is sodium hyaluronate.

8. The agent for sentinel lymph node detection according to claim 1, wherein the weight-average molecular weight of said hyaluronic acid component is from 600,000 to 6,000,000 daltons.

9. The agent for sentinel lymph node detection according to claim 1, wherein the weight-average molecular weight of said hyaluronic acid component is from 1,900,000 to 6,000,000 daltons.

10. A method for detecting a sentinel lymph node, comprising administering a mixed component of a coloring component and a hyaluronic acid component around primary tumor site of a cancer patient.

11. The method for detecting a sentinel lymph node according to claim 10, wherein said mixed component of the coloring component and the hyaluronic acid component is in the form of an aqueous solution.

12. The method for detecting a sentinel lymph node according to claim 10, wherein said mixed component of the coloring component and the hyaluronic acid component is formulated as an injectable formulation.

13. The method for detecting a sentinel lymph node according to claim 10, wherein said coloring component has a dark color.

14. The method for detecting a sentinel lymph node according to claim 10, wherein said coloring component is in a microparticulate form.

15. The method for detecting a sentinel lymph node according to claim 10, wherein said coloring component is a blue colorant, a green colorant, a magnetic iron material, an iron colloid, or colored microparticles.

16. The method for detecting a sentinel lymph node according to claim 10, wherein said hyaluronic acid component is sodium hyaluronate.

17. The method for detecting a sentinel lymph node according to claim 10, wherein the weight-average molecular weight of said hyaluronic acid component is from 600,000 to 6,000,000 daltons.

18. The method for detecting a sentinel lymph node according to claim 10, wherein the weight-average molecular weight of said hyaluronic acid component is from 1,900,000 to 6,000,000 daltons.

19. Use of hyaluronic acid for the manufacture of an agent for sentinel lymph node detection, wherein a main component of said agent is a mixed component of a coloring component and a hyaluronic acid component.
